(19) Europäisches Patentamt

European Patent Office

Office européen des brevets

(11) **EP 2 666 425 B1**

(12) **EUROPEAN PATENT SPECIFICATION**

(45) Date of publication and mention
of the grant of the patent:
**15.05.2019 Bulletin 2019/20**

(51) Int Cl.:
***A61B 18/14*** *(2006.01)*

(21) Application number: **13168721.2**

(22) Date of filing: **22.05.2013**

(54) **Computer-implemented temperature based ablation completeness algorithm**

Computerimplementierter temperaturbasierter Ablationsvollständigkeitsalgorithmus

Algorithme de complétude d'ablation fondée sur la température mise en oeuvre par ordinateur

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.05.2012 US 201213477307**

(43) Date of publication of application:
**27.11.2013 Bulletin 2013/48**

(73) Proprietor: **Covidien LP
Mansfield, MA 02048 (US)**

(72) Inventor: **Ladtkow, Casey M.
Erie, CO 80516 (US)**

(74) Representative: **Soames, Candida Jane et al
Maschio & Soames IP Limited
30 Carlton Crescent
Southampton SO15 2EW (GB)**

(56) References cited:
**EP-A1- 2 499 982      WO-A2-02/054941
US-A1- 2008 183 165    US-B1- 6 575 969**

## Description

## BACKGROUND

Technical Field

The present disclosure relates to energy delivery feedback systems and, more particularly to a system and an exemplary method for determining ablation completeness using temperature.

Background of Related Art

The use of electrical energy including radiofrequency and microwave energy ("RF & MW energy") and, in particular, radiofrequency electrodes or microwave antennae ("RF-electrodes/MW-antennae") for ablation of tissue in the body or for the treatment of pain is known. Generally, such RF electrodes (e.g., probes, resistive heating elements and the like) include an elongated cylindrical configuration for insertion into the body to target tissue which is to be treated or ablated. The RF electrodes can further include an exposed conductive tip portion and an insulated portion. The RF electrodes can also include a method of internal cooling (e.g., a Cool-tip™ or the like), such as the RF electrodes shown and described in U.S. Patent Nos. 6,506,189 entitled "COOL-TIP ELECTRODE THERMOSURGERY SYSTEM" issued to Rittman, III et al., on Jan. 14, 2003 and 6,530,922 entitled "CLUSTER ABLATION ELECTRODE SYSTEM" issued to Cosman et al., on Mar. 11, 2003. Accordingly, when the RF electrode is connected to an external source of radiofrequency power, e.g., an electrosurgical generator (device used to generate therapeutic energy such as radiofrequency (RF), microwave (MW) or ultrasonic (US)), and current is delivered to the RF electrode, heating of tissue occurs near and around the exposed conductive tip portion thereof, whereby therapeutic changes in the target tissue, near the conductive tip, are created by the elevation of temperature of the tissue.

In some applications, for example, tumor ablation procedures, multiple electrodes are inserted into the body in an array to enlarge ablation volumes.

In a particular application, arrays of high frequency electrodes are inserted into tumors. The electrodes are typically placed in a dispersed fashion throughout the tumor volume to cover the tumor volume with uniform heat. The multiple electrodes may be activated simultaneously or sequentially applied with high frequency energy so that each electrode heats the surrounding tissue. During series activation, energy is applied to each electrode one at a time. This sequence of cycling the energy through the electrodes continues at a prescribed frequency and for a period of time.

The electrode systems noted above are limited by the practical size of lesion volumes they produce. Accordingly, electrodes with cooled conductive tips have been proposed. A need still exists, however, for improved ablation systems and methods which provide more feedback during use. In particular, improved systems and methods are needed to inform the surgeon when ablation is complete.

Reference is made to US 2008/0183165 A1, which discloses a thermal feedback system for use during electrosurgical procedures. Reference is also made to US 6,575,969 B1, which discloses a radiofrequency thermosurgery electrode system for tumor ablation.

## SUMMARY

In the drawings and in the description which follows, the term "proximal", as is traditional, will refer to the end of the system, or component thereof, which is closest to the operator, and the term "distal" will refer to the end of the system, or component thereof, which is more remote from the operator.

The present disclosure relates to a system and further an exemplary method for determining ablation completeness using temperature.

According to the present invention, there is provided a system according to the appended independent claim 1. Further preferred embodiments are disclosed in the dependent claims.

According to an aspect of the present disclosure, a system for determining completeness of an ablation procedure on a target tissue includes an electrosurgical energy source and an electrode probe assembly connected to the electrosurgical source. The electrode probe assembly including an electrode assembly having a needle configured to deliver electrosurgical energy to the target tissue. The system further includes a thermal feedback assembly connected to the electrosurgical source and the thermal feedback assembly includes at least one temperature sensor assembly. The system also includes a computer configured to (1) measure a time of energy delivered to the target tissue, (2) receive a temperature reading from the thermal feedback assembly, (3) estimate a size of an ablation volume based on the temperature reading, a distance between the electrode probe assembly and each temperature sensor assembly, and the measured time, and optionally (4) calculate a growth rate of the ablation volume based on the estimated size.

According to a further aspect of the present disclosure, the system may further include a hub configured to selectively support the electrode probe assembly and the thermal feedback assembly, wherein the needle of the electrode probe assembly and each temperature sensor assembly are positioned at a known distance apart.

The temperature sensor assembly may be spaced radially from the electrode probe assembly. The temperature sensor assembly may comprise a plurality of temperature sensors mutually spaced radially apart.

According to another aspect of the present disclosure, the thermal feedback assembly may include a plurality of temperature sensors, and the computer may estimate the size of the ablation based on the temperature at each sensor and distance between each temperature sensor from the electrode probe assembly.

According to a further aspect of the present disclosure, the computer may be further configured to determine the ablation procedure is complete when the growth rate is less than or equal to a threshold.

According to another aspect of the present disclosure, the computer may be configured to interpolate one or more temperatures based on boundary conditions and a single measured temperature. The computer may also determine a temperature and distance from the electrode assembly that indicates that if a growth rate threshold has been reached.

According to another aspect of the present disclosure, a method for determining completeness of an ablation procedure on a target tissue includes the step of inserting an electrode probe assembly and a temperature sensor of a thermal feedback assembly into a patient proximate the target tissue. The method further includes the step of supplying electrosurgical energy to the target tissue via the electrode probe. Also, the method includes the steps of measuring a time of electrosurgical energy delivery to the target tissue and measuring a temperature at a known distance from the electrode probe assembly. Further, the method includes the steps of estimating a temperature of an ablation volume based on the measured temperature, the known distance from the electrode probe assembly, and the measured time of energy delivery, and optionally calculating a growth rate of the ablation volume based on the estimated size.

The method may further include the step of determining the ablation procedure is complete when the growth rate is less than or equal to a threshold.

Alternatively or in addition, the method may further include the step of interpolating one or more temperatures based on boundary conditions and a single measured temperature.

Alternatively or in addition, the method may further include the step of determining a temperature and distance from the electrode assembly that indicates that the growth rate threshold is reached.

Alternatively or in addition, the method may include the step of storing one or more calculation methods within an electrosurgical generator.

Alternatively or in addition, the size of the ablation volume may be estimated using an Arrhenius model calculation on the information received from each thermal feedback assembly.

Alternatively or in addition, the size of the ablation volume may be estimated by performing a tissue damage integral, the integral being over time and including a parameter of tissue temperature varying with time. The integral may also include a parameter of energy applied to tissue.

Alternatively or in addition, the method may include the step of selecting a characteristic energy value to be delivered to the electrode probe assembly based on characteristics of the electrode probe assembly and characteristics of the target tissue to be treated.

Alternatively or in addition, the method may include the step of providing a hub to hold the electrode probe assembly and the thermal feedback assembly. Alternatively, the hub may be configured to hold the electrode probe assembly and a plurality of thermal feedback assemblies, wherein each thermal feedback assembly is held at a known distance from the electrode probe assembly.

Alternatively or in addition, the method may include the step of estimating the size of the ablation volume based on the plurality of measured temperatures and the known distances from the electrodes.

According to another aspect of the present disclosure, a method for determining completeness of an ablation procedure on a target tissue includes the steps of measuring a time of electrosurgical energy delivery to target tissue and measuring a temperature at a known distance from an electrode probe assembly. The method further includes the steps of estimating a temperature of an ablation volume based on the measured temperature, known distance from the electrode probe assembly, and the measured time of energy delivery and optionally calculating a growth rate of the ablation volume based on the estimated size.

The method may further include the steps of interpolating one or more temperatures at respective distances from the electrode probe assembly optionally based on boundary conditions. The one or more temperatures may be interpolated using a logarithmic decay calculation. The method may include determining the ablation volume based on determining a distance from the electrode probe assembly where a tissue damage threshold is reached. The tissue damage threshold is compared to a tissue damage integral parameter involving integrating a temperature parameter over time in determining the ablation volume, wherein the tissue damage integral parameter varies with the distance from the electrode probe assembly.

Alternatively or in addition, the method may include the steps of measuring more than one temperature, interpolating one or more temperatures and distances from the electrode probe assembly based on the more than one temperature measured. The method may include determining the ablation volume based on determining a distance from the electrode probe assembly where a tissue damage threshold is reached.

In a generally applicable embodiment, a tissue damage parameter is determined based on the measured temperature and the measured time, wherein a damage parameter threshold is used in conjunction with tissue damage parameter values that vary with the distance from the electrode probe assembly to determine the ablation volume where the damage parameter exceeds the damage parameter threshold.

These and other aspects and advantages of the disclosure will become apparent from the following detailed description and the accompanying drawings, which illustrate by way of example the features of the disclosure.

## BRIEF DESCRIPTION OF THE DRAWINGS

Various embodiments of the present disclosure are described herein with reference to the drawings wherein:

Fig. 1 is a schematic illustration of one embodiment of a thermal feedback system of the present disclosure operatively associated with a target surgical site;

Fig. 2 is an illustration of the electrode probe assembly and the thermal feedback assembly of Fig. 1;

Fig. 3 is a schematic illustration of a further embodiment of a thermal feedback system operatively associated with a target surgical site;

Fig. 4A is an illustration of the thermal feedback assembly of Fig. 3;

Fig. 4B is an illustration of a thermal feedback assembly according to an embodiment of the present invention;

Fig 5 is a plot of temperature with respect to distance from an electrode according to an embodiment of the present disclosure;

Figs. 6A-6C are plots of temperature with respect to distance from an electrode according to an embodiment of the present disclosure; and

Fig. 7 is a flow chart of a method according to an embodiment of the present disclosure.

## DETAILED DESCRIPTION OF EMBODIMENTS

The systems and methods of the present disclosure provide for a more precise controlled monitoring and/or feedback of an electrode probe during therapeutic use in a target surgical site, e.g., in a cancer tumor. Moreover, the systems and methods of the present disclosure provide for an improved ability to predict and/or estimate the depth and/or volume of treatment possible by the electrode probe when the electrode probe of an electrosurgical treatment device is set to a particular or various operative parameters.

It will be readily apparent to a person skilled in the art that the systems and methods of use of the systems can be used to monitor or provide feedback during treatment of body tissues in any body cavity or tissue locations that are accessible by percutaneous or endoscopic catheters or open surgical techniques, and is not limited to cancer tumors or the like. Application of the systems and methods in any corporal organ and/or tissue is intended to be included within the scope of the present disclosure.

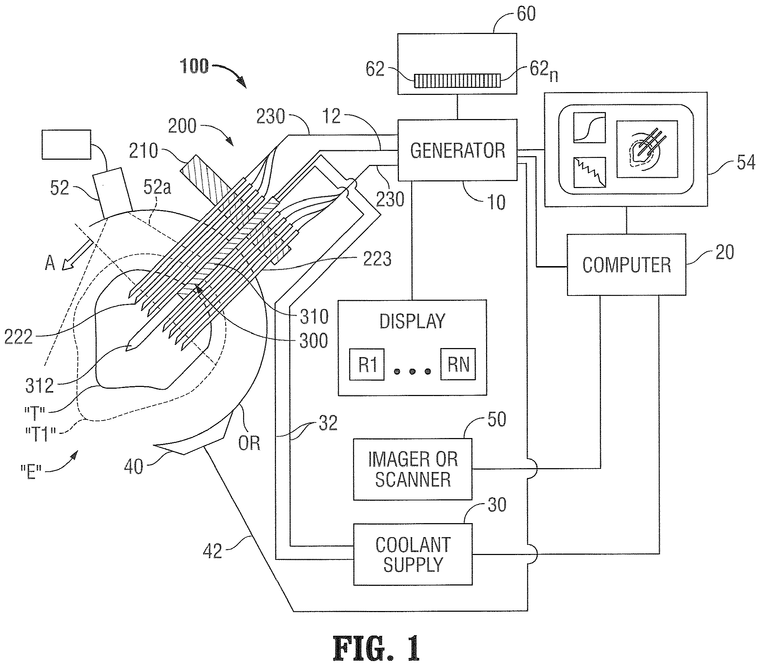

With reference to Fig. 1, a thermal feedback system, according to an embodiment of the present disclosure, is generally designated as 100. Feedback system 100 includes a thermal feedback assembly 200 operatively connected to an electrosurgical generator and/or energy source 10 and/or computer 20.

At least one electrode probe assembly 300 is provided which is operatively associated with feedback assembly 200 and is connectable to electrosurgical energy source 10 in order to perform tissue ablation and the like. Each electrode probe assembly 300 may include a rigid shaft, antenna or needle 310 configured for insertion into a target tissue or organ "OR". Needle 310 of each probe assembly 300 may terminate in an exposed distal tip 312 having a pointed configuration for facilitating percutaneous insertion of needle 310 into body organ "OR". A portion of the external surface of needle 310 of each electrode probe assembly 300 is covered with an insulating material, as indicated by hatched line areas in Fig. 1. Distal tip 312 remains uncovered and is connected, through needle 310, to cable 12 and thereby to electrosurgical energy source 10. Electrode probe assembly 300 may include a coolant supply 30 fluidly connected to needle 310 for circulating a fluid thereto via conduit(s) 32.

Reference may be made to commonly assigned U.S. Application Serial No. 11/495,033, filed on July 28, 2006, published as US 2008-0027424 A1, and entitled "COOL-TIP THERMOCOUPLE INCLUDING TWO-PIECE HUB" for a detailed discussion of the construction and operation of electrode probe assembly 300.

Temperatures at or near the exposed distal tip(s) 312 of needle(s) 310 may be controlled by adjusting a flow of fluid coolant through needle 310. Accordingly, the temperature of the tissue contacting at or near distal tip(s) 312 is controlled. In operation, fluid from coolant supply 30 is carried the length of needle 310 through an inner tube (not shown) extending therethrough to the distal end of needle 310 terminating in an open end or cavity (not shown) of distal tip 312. At the opposite end of needle 310, the inner tube is connected to receive fluid. Backflow from distal tip(s) 312 is through an exit port (not shown) of needle 310.

Feedback system 100 may further include a reference electrode 40 that may be placed in contact with the skin of a patient or an external surface of organ "OR" with a connection 42 to electrosurgical energy source 10. Reference electrode 40 and connection 42 serve as a path for return current from electrosurgical energy source 10 through needle 310 of electrode probe assembly 300.

As can be seen in Fig. 1, by way of illustration only, a targeted region to be ablated is represented in sectional view by the line "T". It is desired to ablate the targeted region "T" by fully engulfing targeted region "T" in a volume of lethal heat elevation. The targeted region "T" may be, for example, a tumor which has been detected by an image scanner 50. For example, CT, MRI, fluoroscopy or ultrasonic image scanners may be used, and the image data transferred to computer 20. As an alternative example, an ultrasonic scanner head 52 may be disposed in contact with organ "OR" to provide an image illustrated by lines 52a.

For example, in Fig. 1, dashed line "T1" represents the ablation isotherm in a sectional view through organ "OR". Such an ablation isotherm may be that of the surface achieving possible temperatures of approximately 50°C or greater. At that temperature range, sustained for approximately 30 seconds to approximately several minutes, tissue cells will be ablated. The shape and size of the ablation volume, as illustrated by dashed line "T1", may accordingly be controlled by a configuration of the electrode probe assemblies 300 used, the geometry of distal tips 312 of electrode probe assemblies 300, the amount of RF power applied, the time duration that the power is applied, the cooling of the needles 310 of electrode probe assemblies 300, etc.

Data processors may be connected to display devices to visualize targeted region "T" and/or ablation volume "T1" in real time during the ablation procedure.

As illustrated in Fig. 1, feedback system 100 may further include a library 60 including a plurality of thermal profiles/overlays $62_n$. As used herein, the term library is understood to include and is not limited to repository, databank, database, cache, storage unit and the like. Each overlay 62 includes a thermal profile which is characteristic of and/or specific to a particular configuration of cannula/electrode assembly or amount of exposure (i.e., specific to the length of exposure of distal tip 312 of needle 310 or the amount of needle 310 extending from a distal tip of a cannula) of the cannula/electrode assembly. In addition, for each amount of exposure or configuration of the cannula/electrode assembly, a plurality of overlays $62_n$ is provided which includes a thermal profile which relates to, for example, the amount of time electrode probe assembly 300 is activated, to the temperature to which electrode probe assembly 300 is heated, etc.

With continued reference to Fig. 1, feedback system 100, as mentioned above, includes a thermal feedback assembly 200 operatively connected to an electrosurgical generator 10 and/or computer 20. Thermal feedback assembly 200 is operatively associated with the at least one electrode probe assembly 300.

As shown in Fig. 1, feedback assembly 200 includes a hub or housing 210 configured to selectively support at least one electrode probe assembly 300 and at least one temperature sensor assembly 220. A plurality of temperature sensor assemblies 220 are shown supported in housing 210 on opposed sides of a single electrode probe assembly 300. It is contemplated that any number of temperature sensor assemblies 220 may be disposed on a single side, on opposed sides, or on multiple sides of the single electrode probe assembly 300 or relative to multiple electrode probe assemblies 300. It is further contemplated that multiple temperature sensor assemblies 220 may be interspersed amongst multiple electrode probe assemblies 300. Individual needles, cannulae or introducers 223 may be used to introduce temperature sensors 222 into the target site or organ "OR".

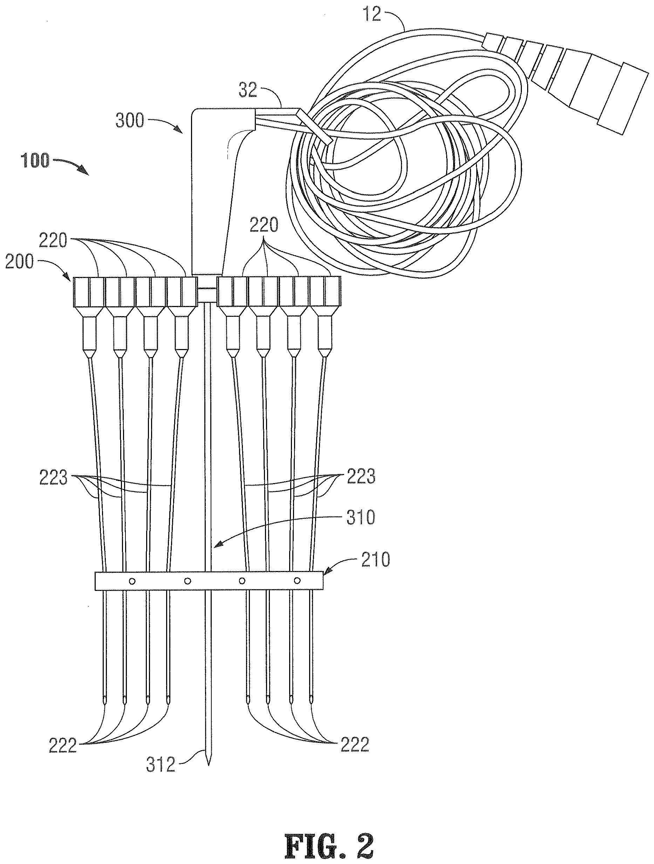

As shown in Fig. 2, housing 210 is used to position temperature sensor assemblies 220 on opposed sides of a singe electrode probe assembly 300 so as to define a single axis or plane. Housing 210 may be configured to position cannulae 223 and temperature sensors 222 of temperature sensor assemblies 220 at a known distance from electrode probe assembly 300 and/or from one another, or are equi-distant or uniformly spaced from one another.

Each temperature sensor assembly 220 is electrically or optically connected to electrosurgical generator 10 via a suitable electrical connector or the like 230.

Temperature sensors 222 include one or more of an emitter, sensor or marker to provide special relationship to electrode assembly 310. Each temperature sensor assembly 220 may include a temperature sensor 222 in the form of a rigid or semirigid cannula 223 and/or needles configured for insertion and/or penetration into the target surgical site. Suitable temperature sensors 222 may include thermocouples, resistive temperature devices (RTD) or fiber optic temperature probes. One example of a temperature sensor is sold under the tradename "Fluoroptic® Thermometer, available from Luxtron®, Santa Clara, CA. Such sensor types are configured to measure the decay time of light emitted from phosphorescent materials (e.g. phosphors). The decay time is a persistent property of the sensor that varies directly with the temperature. Additionally, temperature sensors are shown and described in U.S. Patents 4,075,497; 4,215,275; 4,448,547; 4,560,286; 4,752,141; 4,883,354; and 4,988,212.

Other suitable temperature sensors for use with temperature sensor assemblies 220, to measure the temperature at a target surgical site, include and are not limited to optical sensors (e.g., Flouroptic®, infrared, etc.), thermocouples, Resistance-Temperature-Detectors (RTD), thermistors, MRI, fluoroscopic, ultrasound, CT, radiometry and the like.

Temperature sensors 222 may be configured to measure or monitor temperatures greater than about 60°C. In an embodiment, feedback system 100 may be provided with suitable algorithms or the like for interpolating temperature values from at least two temperature sensors 222 and/or for integrating thermal damage from at least two temperature sensors 222. One real-time temperature sensor may be used in conjunction with an assumed or predetermined value from a look-up table or similar method.

The temperature measurements delivered to feedback system 100 may be used to generate a thermal map of the target area and/or, upon integration, may be used to account for particular tissue characteristics, such as, for example, perfusion, conduction, resistance and/or density.

In an embodiment, temperature sensors 222 may be deployed around needle 310 of the electrode probe assembly 300. Such temperature sensors may be constructed of suitable shape memory alloys so as to permit the temperature sensor to wrap around needle 310. Additionally, in an embodiment, a cannula including temperature sensors may be deployed about needle 310 of the electrode probe assembly 300.

Electrosurgical generator 10 and electrode probe assembly 300 may be configured to deliver energy to at least one of a radiofrequency, a microwave, an ultrasound, and a cryo-therapy needle.

Feedback system 100 is capable of providing size predictability for ablation volume to be created during a thermal procedure of a target region prior to the ablation volume exceeding a predetermined volume during the thermal procedure. For example, feedback system 100 may provide feedback regarding a volume of the thermal therapy (e.g., diameter), and estimation of an overall size of the volume of the thermal therapy, an estimation of a rate of growth of the volume of the thermal therapy, and/or an estimation of a time to completion of the thermal therapy. All of this information may be displayed on a monitor 54 (See Fig. 1) or the like. Additionally, monitor 54 may illustrate the growth of the ablation volume, in real-time, as the procedure is going forward.

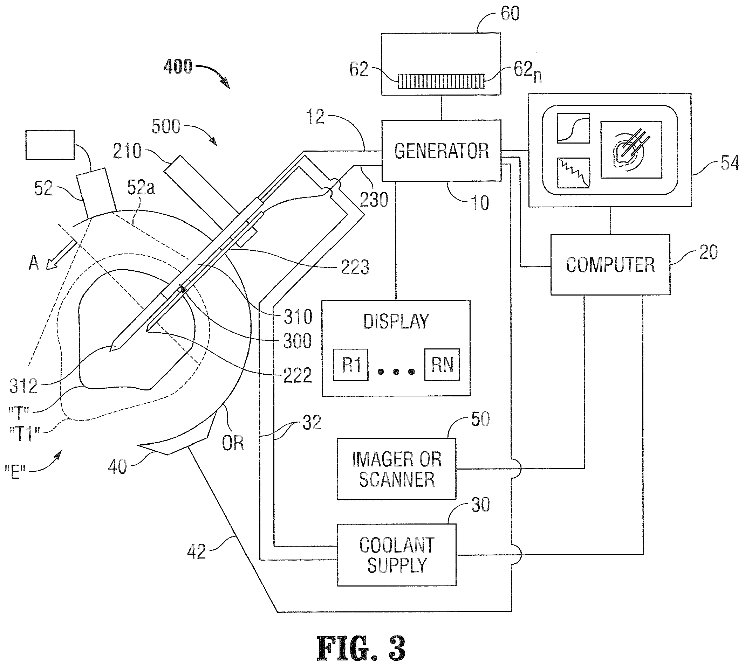

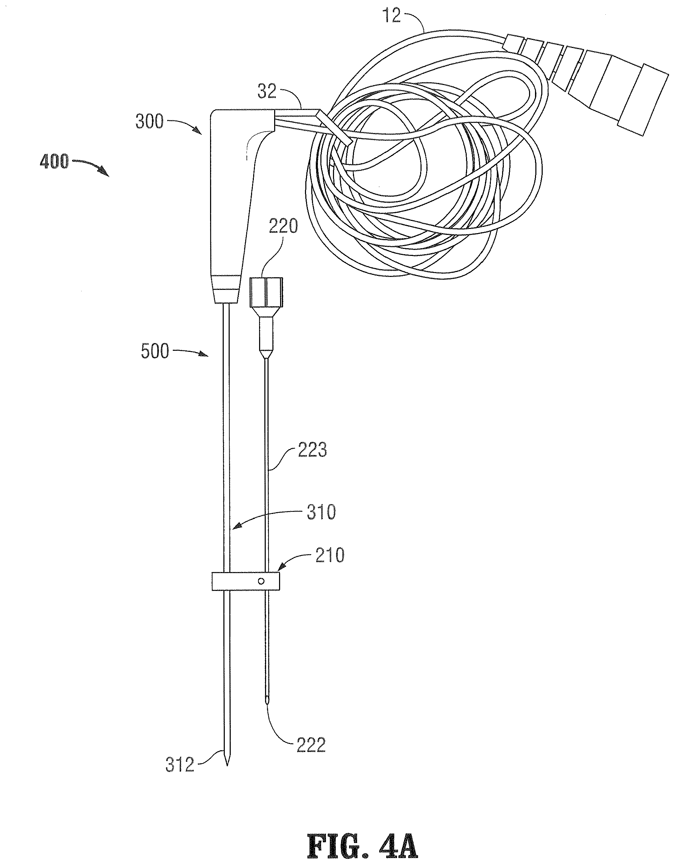

Figs. 3 and 4A show an alternative embodiment of a feedback system 400 including temperature feedback assembly 500. For clarity purposes and to avoid unnecessary repetition, the electrosurgical generator and/or energy source and/or computer included in the system as noted above in the description of the embodiment of Fig. 1 are not shown. Temperature feedback assembly 500 includes hub or housing 210 configured to selectively support one electrode probe assembly 300 and one temperature sensor assembly 220. It is contemplated that the temperature sensor assembly 220 may be disposed on either side the single electrode probe assembly. Similar to Fig. 1, individual needles, cannulae or introducers 223 may be used to introduce temperature sensor 222 into the target site or organ "OR".

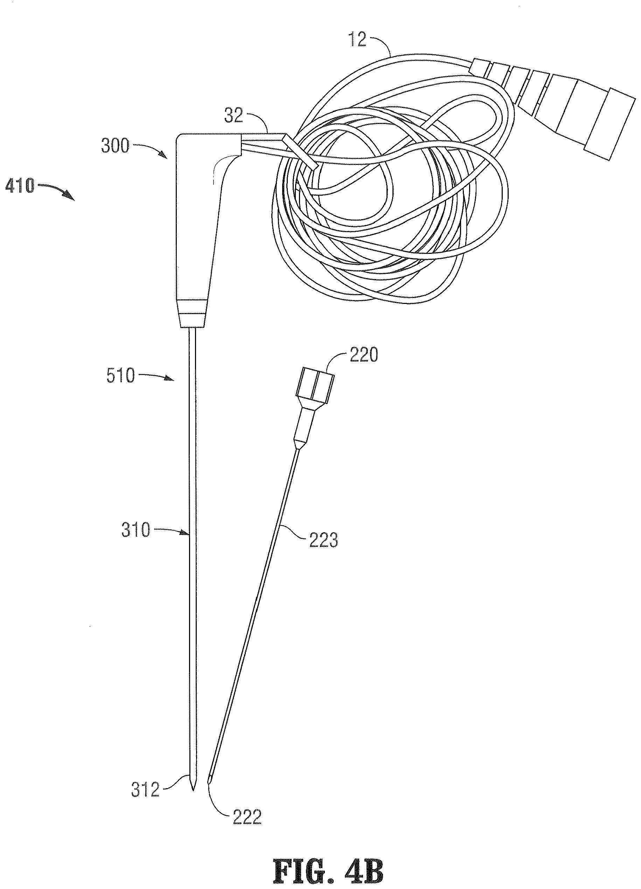

Fig. 4B shows an alternative feedback system 410 including temperature feedback assembly 510. Temperature feedback assembly 510 includes one electrode probe assembly 300 and one temperature sensor assembly 220. It is contemplated that the temperature sensor assembly 220 may be disposed on either side of the single electrode probe assembly 300. The temperature sensor assembly 220 may be inserted at any angle or distance relative to the electrode probe assembly 300. An ultrasound, CT, or other similar imaging technology is used to measure the distance between the temperature sensor 222 and distal tip 312 of the electrode probe assembly 300. Similar to Fig. 1, individual needles, cannulae or introducers 223 may be used to introduce temperature sensor 222 into the target site or organ "OR".

A method of the present disclosure includes determining completion of an ablation procedure based on measured time and measured temperature. The method may comprise the step of measuring a temperature of the target tissue or organ "OR", at known distances relative thereto, during and/or post treatment of the target tissue or organ "OR". The temperature of the target tissue or organ "OR", at the known distance, may be an absolute temperature and/or a temperature that is interpolated. Additionally, the method may comprise integrating the temperature over time to determine an extent of thermal treatment. Such an integration may be calculated using an "Arrhenius thermal treatment integral" or other methods of thermal damage estimation.

As used herein, "thermal damage" is a term that describes a quantity representing a relative amount of destruction to a tissue component. The component of interest can vary widely between applications from subcellular components, such as, for example, protein or organelles, to many celled systems, such as, for example, tumors or organs. To study systems spanning such a wide range of scale different techniques may be applied. For a relatively small system, one approach may be an "ab initio" method or some other molecular dynamic approach. For relatively larger systems, one approach may be to use an empirical method, such as, for example, the "Arrhenius" method described herein or a critical temperature criterion.

The term "Arrhenius thermal treatment" refers to a method of quantifying thermal effects on underlying tissue. The present method thus models microscopic effects in tissue, such as, for example, the denaturation of a single species of protein, or models macroscopic effects in tissue, such as, for example, a color change of the tissue associated with the thermal treatment where many different reactions have taken place.

The equation for the "Arrhenius model" may be represented by the following equation:

$$\Omega = -\ln\left(\frac{C(\tau)}{C(0)}\right) = A\int_{0}^{\tau} e^{\left(\frac{E}{RT(t)}\right)} dt$$

where:

    $\Omega$ = is the thermal effect sustained by the tissue or organ (tissue damage or damage intergral);
    $C(\tau)$ = is the concentration as a function of specific time, $\tau$;
    $C(0)$ = is the initial concentration at time zero;
    $A$ = is the frequency factor, approximately $7.39 \times 10^{39}$ l/s (specific to liver tissue);
    $\Delta E$ = is the activation energy, approximately $2.577 \times 10^{5}$ J/mol (specific to liver tissue);
    $R$ = is the ideal gas constant; and
    $T(t)$ = is the temperature as a function of time variable, t.

The "Arrhenius model" is used because, in addition to combined processes, the "Arrhenius model" applies to individual processes as well. Individual processes that may be of interest include and are not limited to the denaturation of a lipid bi-layer of a cell, the denaturation of mitochondrial proteins, and the denaturation of nuclear proteins. The denaturation of lipid bi-layer is of interest because the lipid bi-layer loses its structure before many other parts of a cell. The denaturation of mitochondrial and nuclear proteins is of interest because they denature at temperatures in the range of about 42 to 60°C.

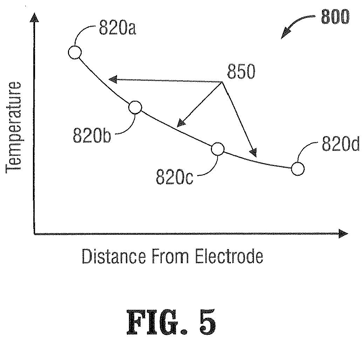

The plot 800 shown in Fig. 5 visualizes the method of correlating a plurality of measured temperatures 820a-820d (See feedback assembly 200 in Figs. 1 and 2) with distance from electrodes and interpolating a curve based on connecting (lines 850) the measured temperature values 820a-820d.

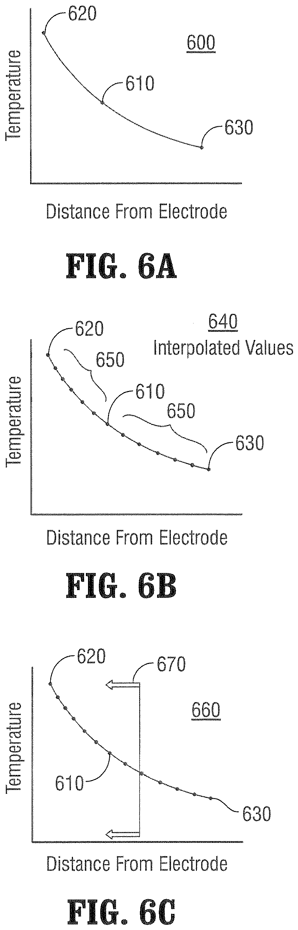

A relationship of temperature and ablation size is shown in plots 600, 640 and 660 of Figs. 6A-C, respectively when using a temperature feedback assembly 500 having only a single temperature sensor assembly 220. Plot 600 shows a temperature graph with boundary conditions 620, 630 applied to the temperature measurements. Generally, the plot 600 can be interpolated with a single temperature measurement 610 at a known distance. Boundary conditions 620, 630 represent the outer edges of the ablation volume. The boundary conditions 620, 630 may be about 100°C at about 0.5 cm from the electrode assembly and about 37.5°C (about average body temperature or average temperature of a particular organ) at about 3 cm, respectively.

Then one or more interpolated values 650 may be calculated as shown in plot 640. The interpolated values are calculated using a logarithmic decay equation. Line 670 of plot 660 shows at what temperature and distance from the electrode that the threshold is reached. When the damage integral ($\Omega$) is about 4.6 (threshold) then about 99.9% cell death is reached.

The plots 600, 640 and 660 visualize the method for correlating the temperature with distance from electrodes and utilize a logarithmic fit to approximate the temperature field based on a single measured temperature 610. The damage integral ($\Omega$) is used to estimate the damage done to the tissue.

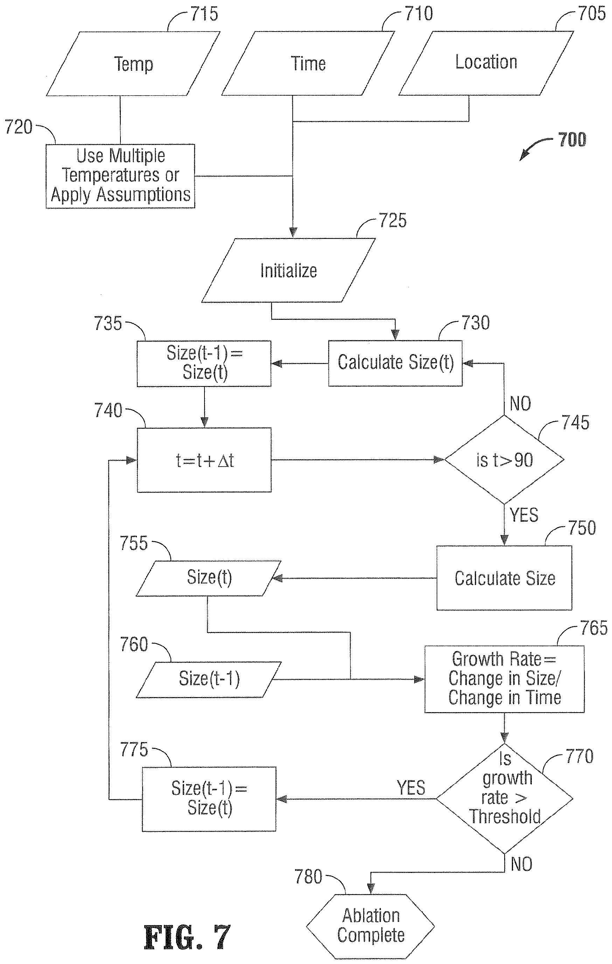

Fig. 7 shows a method 700 for determining ablation completion based on time and temperature, which are measured by the generator 10. The method measures the location at block 705 and time at block 710. The method also measures the temperature at block 715. The temperature measured may be a single temperature with boundary conditions applied or a plurality of temperatures measured at block 720.

The algorithm is initialized at block 725 and the formulas for calculating the size and growth rate are preloaded. At block 730, a current size is calculated using a rate type calculation (e.g., first order rate calculation) based on the preloaded formulas which are based on the plots of Figs 6A-C and Arrhenius model formula, as described above.

The current size is also saved as previous size at block 735 and time is incremented by a desired interval at block 740. Then at block 745, current time is then compared with an initial time threshold corresponding to the point of time at which temperature, time and size begin correlating. The algorithm utilizes a period of about 90 seconds. In embodiments, the period may be any suitable interval selected based on a variety of tissue and energy parameters.

Once the initial period of time has expired, the algorithm begins to calculate and compare the growth rate at block 750.

$$GrowthRate = \frac{Size(t) - Size(t-1)}{Time(t) - Time(t-1)}$$

In particular, the method calculates the size of the ablation volume and saves the value as the current size at block 755. The current size is then used in conjunction with the previously calculated size (block 760) to determine the growth rate via differentiation at block 765. Next at block 770, if the growth rate is below the predetermined threshold, the current size is saved as previous size at block 775 and the method returns to the time incrementation step (block 740) to repeat the size and growth rate calculations. If the growth rate is above the threshold, the method deems the ablation to be complete at block 780, at which point the generator 10 may issue an alarm and/or terminate the energy supply.

While the above description contains many specific examples, these specific should not be construed as limitations on the scope of the disclosure, but merely as exemplifications of preferred embodiments thereof.

**Claims**

1. A system (100) for determining completeness of an ablation procedure on a target tissue, the system comprising:

   an electrosurgical energy source (10);
   a single electrode probe assembly (300) connected to the electrosurgical energy source, the electrode probe assembly including a needle (310) configured to deliver electrosurgical energy to the target tissue;
   a thermal feedback assembly (200) connected to the electrosurgical source, the thermal feedback assembly including a plurality of temperature sensor assemblies (220) located on opposed sides of the single electrode probe assembly so as to define a single axis or plane; and
   a computer (20) configured to:

      (1) measure a time of energy delivered to the target tissue,

(2) receive a temperature reading from the thermal feedback assembly,

(3) estimate a size of an ablation volume based on the temperature reading, a distance between the electrode probe assembly and each temperature sensor assembly of the plurality of temperature assemblies, and the measured time, and

(4) calculate a growth rate of the ablation volume based on a previous and a current estimated size,

wherein the computer is further configured to determine the ablation procedure is complete when the growth rate is less than or equal to a growth rate threshold, the system being arranged such that when the ablation is deemed to be complete, the electrosurgical energy source (10) terminates the energy supply.

2. The system according to claim 1, wherein the thermal feedback assembly further comprises a hub (210) configured to selectively support the electrode probe assembly and the at least one temperature sensor assembly, wherein the needle of the electrode probe assembly and each temperature sensor assembly of the at least one temperature sensor assembly are positioned at a known distance apart.

3. The system according to claim 1 or 2, wherein the thermal feedback assembly includes a plurality of temperature sensors (222), and the computer estimates the size of the ablation based on the temperature at each sensor and the distance between each temperature sensor from the electrode probe assembly.

4. The system according to any preceding claim, wherein the computer interpolates one or more temperatures based on boundary conditions (620, 630) and a single measured temperature.

5. The system according to any preceding claim, wherein the computer determines a temperature and distance from the electrode assembly that indicates if a growth rate threshold has been reached.

**Patentansprüche**

1. System (100) zum Bestimmen der Vollständigkeit eines Ablationsverfahrens an einem Zielgewebe, wobei das System umfasst:

eine elektrochirurgische Energiequelle (10); eine einzelne Elektrodensondenanordnung (300), die mit der elektrochirurgischen Energiequelle verbunden ist, wobei die Elektrodensondenanordnung eine Nadel (310) aufweist, die konfiguriert ist, um elektrochirurgische Energie an das Zielgewebe zu liefern;

eine thermische Feedbackanordnung (200), die mit der elektrochirurgischen Quelle verbunden ist, wobei die thermische Feedbackanordnung eine Vielzahl von Temperatursensoranordnungen (220) aufweist, die sich auf gegenüberliegenden Seiten der einzelnen Elektrodensondenanordnung befinden, um eine einzelne Achse oder Ebene zu definieren; und

einen Computer (20), der konfiguriert ist zum:

(1) Messen einer Zeit der Energiezufuhr zum Zielgewebe,

(2) Empfangen einer Temperaturmessung von der thermischen Feedbackanordnung,

(3) Schätzen einer Größe eines Ablationsvolumens basierend auf der Temperaturmessung, einem Abstand zwischen der Elektrodensondenanordnung und jeder Temperatursensoranordnung der Vielzahl von Temperaturanordnungen und der gemessenen Zeit, und

(4) Berechnen einer Wachstumsrate des Ablationsvolumens basierend auf einer vorherigen und einer aktuellen geschätzten Größe,

wobei der Computer weiter konfiguriert ist, um zu bestimmen, dass das Ablationsverfahren vollständig ist, wenn die Wachstumsrate kleiner oder gleich einer Wachstumsratenschwelle ist, wobei das System eingerichtet ist, so dass, wenn die Ablation als vollständig gilt, die elektrochirurgische Energiequelle (10) die Energiezufuhr beendet.

2. System nach Anspruch 1, wobei die thermische Feedbackanordnung weiter eine Nabe (210) umfasst, die konfiguriert ist, um die Elektrodensondenanordnung und die mindestens eine Temperatursensoranordnung selektiv zu tragen, wobei die Nadel der Elektrodensondenanordnung und jede Temperatursensoranordnung der mindestens einen Temperatursensoranordnung in einem bekannten Abstand zueinander positioniert sind.

3. System nach Anspruch 1 oder 2, wobei die thermische Feedbackanordnung eine Vielzahl von Temperatursensoren (222) aufweist und der Computer die Größe der Ablation basierend auf der Temperatur an jedem Sensor und dem Abstand zwischen jedem Temperatursensor und der Elektrodensensoranordnung schätzt.

4. System nach einem vorstehenden Anspruch, wobei der Computer eine oder mehrere Temperaturen basierend auf Randbedingungen (620, 630) und einer einzigen gemessenen Temperatur interpoliert.

5. System nach einem vorstehenden Anspruch, wobei der Computer eine Temperatur und einen Abstand von der Elektrodenanordnung bestimmt, was anzeigt, ob eine Wachstumsratenschwelle erreicht wurde.

**Revendications**

1. Système (100) de détermination de la complétude d'une procédure d'ablation sur un tissu cible, le système comprenant :

une source d'énergie électrochirurgicale (10) ;
un ensemble de sonde à électrode unique (300) connecté à la source d'énergie électrochirurgicale, l'ensemble de sonde à électrode incluant une aiguille (310) configurée pour délivrer une énergie électrochirurgicale au tissu cible ;
un ensemble de rétroaction thermique (200) relié à la source électrochirurgicale, l'ensemble de rétroaction thermique incluant une pluralité d'ensembles de capteurs de température (220) situés sur des côtés opposés de l'ensemble de sonde à électrode unique de façon à définir un axe ou plan unique ; et
un ordinateur (20) configuré pour :

(1) mesurer un temps d'énergie délivrée au tissu cible,
(2) recevoir un relevé de température de l'ensemble de rétroaction thermique,
(3) estimer une taille d'un volume d'ablation sur la base du relevé de température, une distance entre l'ensemble de sonde à électrode et chaque ensemble de capteur de température de la pluralité d'ensembles de température et le temps mesuré, et
(4) calculer une vitesse de croissance du volume d'ablation sur la base d'une taille précédente et estimée actuellement,

dans lequel l'ordinateur est en outre configuré pour déterminer si la procédure d'ablation est terminée lorsque la vitesse de croissance est inférieure ou égale à un seuil de vitesse de croissance, le système étant agencé de telle sorte que lorsque l'ablation est jugée terminée, la source d'énergie électrochirurgicale (10) met fin à l'alimentation en énergie.

2. Système selon la revendication 1, dans lequel l'ensemble de rétroaction thermique comprend en outre une plate-forme (210) configurée pour supporter sélectivement l'ensemble de sonde à électrode et l'au moins un ensemble de capteur de température, dans lequel l'aiguille de l'ensemble de sonde à électrode et chaque ensemble de capteur de température de l'au moins un ensemble de capteur de température sont positionnés à une distance connue les uns des autres.

3. Système selon la revendication 1 ou 2, dans lequel l'ensemble de rétroaction thermique inclut une pluralité de capteurs de température (222), et l'ordinateur estime la taille de l'ablation sur la base de la température au niveau de chaque capteur et la distance entre chaque capteur de température de l'ensemble de sonde à électrode.

4. Système selon l'une quelconque des revendications précédentes, dans lequel l'ordinateur effectue une interpolation d'une ou plusieurs températures sur la base des conditions limites (620, 630) et d'une température mesurée unique.

5. Système selon l'une quelconque des revendications précédentes, dans lequel l'ordinateur détermine une température et une distance depuis l'ensemble à électrode qui indique si un seuil de vitesse de croissance a été atteint.

FIG. 1

**FIG. 2**

**FIG. 3**

EP 2 666 425 B1

**FIG. 4A**

**FIG. 4B**

FIG. 5

**FIG. 6A**

**FIG. 6B**

**FIG. 6C**

**FIG. 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6506189 B
- US 6530922 B
- US 20080183165 A1
- US 6575969 B1
- US 49503306 A
- US 20080027424 A1
- US 4075497 A
- US 4215275 A
- US 4448547 A
- US 4560286 A
- US 4752141 A
- US 4883354 A
- US 4988212 A